# EUROPÄISCHE PATENTSCHRIFT

(11) **EP 2 190 372 B1**
(45) Veröffentlichungstag und Bekanntmachung des Hinweises auf die Patenterteilung: **08.06.2011**
(21) Anmeldenummer: 08802216.5
(22) Anmeldetag: 15.09.2008
(51) Int. Cl.: A61B 17/88, A61C 8/00, B25B 13/46, B25B 23/142

(54) **SCHRAUBWERKZEUG MIT EINEM RATSCHENKOPF MIT ELASTISCHER UMFASSUNG**
SCREWING TOOL HAVING A RATCHET HEAD WITH AN ELASTIC GRIP
OUTIL DE VISSAGE DOTÉ D'UNE TÊTE À CLIQUET À CERCLAGE ÉLASTIQUE

(30) Priorität: 14.09.2007 DE 202007012866 U; 10.11.2007 DE 202007015689 U; 12.09.2008 DE 102008046989
(43) Veröffentlichungstag der Anmeldung: 02.06.2010
(73) Patentinhaber: Teichmann, Martin, 60439 Frankfurt (DE)
(72) Erfinder: RECK, Bernhard, 35236 Breidenbach (DE); TEICHMANN, Martin, 60439 Frankfurt (DE)
(74) Vertreter: Grünecker, Kinkeldey, Stockmair & Schwanhäusser Anwaltssozietät
(86) Internationale Anmeldenummer: PCT/EP2008/007681
(87) Internationale Veröffentlichungsnummer: WO 2009/036943

(56) Entgegenhaltungen:
- WO-A-99/36229
- DE-A1- 3 721 564
- DE-U1-202004 014 195
- US-A- 5 557 994
- US-A- 6 109 150

## Beschreibung

Die Erfindung betrifft ein Schraubwerkzeug mit einem Ratschenkopf und einem Betätigungsgriff für die Anwendung in der Medizinaltechnik, insbesondere der Dentalprotetik.

Die Zuverlässigkeit von Verschraubungen bei Implantaten bedingt eine genaue Einhaltung von Vorgaben für Vorspannkräfte, wie dem Drehmoment. Hierfür wird in der Regel ein Drehmomentschlüssel benutzt, der in der Regel zur besseren Handhabung bei beengten Platzverhältnissen mit einer Ratsche versehen ist. Eine Ratsche ermöglicht in bekannter Weise das Anziehen einer Verschraubung in möglichst kleinen Winkelschritten. Sie überträgt Schwenkbewegungen eines Hebels in nur einer Richtung, während die andere Richtung gesperrt ist. In der Regel bestehen Ratschen aus mehreren Teilen, wie Ratschenkörper, Federelement, Schlüsselhalter, Ratschrad, Ratschkopf, Fixierstiften und gegebenenfalls mehr. Für eine nötige Sterilisation müssen alle Teile demontiert, sterilisiert und wieder zusammengesetzt werden.

Mit dem Patent DE 19507535 wird eine Ratsche für medizinische Zwecke beschrieben, bei der sich auch ein vorgegebenes Drehmoment einstellen lässt. Diese Drehmomentenratsche weist viele Einzelteile auf und hat Dichtungen, um das Eindringen von Sterilisationsflüssigkeit zu verhindern. Sie entspricht nicht mehr den heutigen Möglichkeiten an die Rückführbarkeit von Montageparametern.

In der Patentschrift EP 0704281 B1 ist ein chirurgischer Drehmomentenschlüssel mit Indikator beschrieben. Er weist am Kopf eine Ratsche auf, die wiederum aus mehreren Einzel- und Hohlteilen besteht. Bei der Sterilisation können Teile abhanden kommen oder beschädigt werden. Im Verlauf der Benutzungszeit kann infolge der Sterilisation eine zunehmende Oberflächenrauhigkeit zu zusätzlicher Reibung und damit zu einer Verfälschung des gewünschten Drehmoments führen.

Mit dem Gebrauchsmuster DE 202004014195 U1 wird eine einteilige Ratsche beschrieben, die für die Federung der Klinke eine Klinkenfeder in einem kanalförmigen Spielraum aufweist. Der Eingriff der gefederten Klinke mit dem Eindrehwerkzeug wird über ein Verklemmen der Klinke realisiert. Das Drehmoment wird über die Verformung eines Biegeastes angezeigt. Anordnung und Formgebung von Klinke und Klinkenfeder sind äußerst filigran, wodurch eine aufwendige Fertigung der Ratsche bedingt ist. Auch die Reinigung und Sterilisation der Ratsche gestaltet sich dadurch schwierig. Teile aus der Umgebung können die Klinke verklemmen bzw. die Funktion der Klinke wesentlich beeinträchtigen. Zudem kann die Anzeige des Drehmoments verfälscht werden.

Ein Schraubwerkzeug gemäß den Merkmalen des Oberbegriffs des Anspruchs 1 ist aus WO-A-9936229 bekannt.

Neben den bereits genannten Nachteilen der aus dem Stand der Technik bekannten Ratschenwerkzeuge ist allen beschriebenen Instrumenten gemein, dass die Herstellkosten hoch sind, das Ergebnis eines in die Werkzeuge integrierten bzw. mit den Werkzeugen kombinierten Drehmomentanzeigegeräts verfälscht werden kann und sich die Reinigung und Sterilisation der Werkzeuge aufwendig gestaltet.

Aufgabe der Erfindung ist deshalb eine wesentliche Vereinfachung des Ratschenkopfes, um eine einfachere Reinigung und Sterilisation bei gleichzeitig geringeren Herstellkosten zu erreichen. Zudem soll die Handhabbarkeit bei beengten Platzverhältnissen weiter verbessert werden. Außerdem soll sichergestellt werden, dass das Ergebnis eines integrierten oder in Kombination verwendeten Drehmomentanzeigegeräts nicht verfälscht wird.

Die Aufgabe wird erfindungsgemäß durch die Merkmale des Anspruchs 1 gelöst. Es wird ausgegangen von einem Schraubwerkzeug mit einem Ratschenkopf und einem Betätigungsgriff, wobei der Ratschenkopf eine Aufnahmeöffnung zum Einsetzen eines herkömmlichen, eine im Wesentlichen zylindrische Handhabe mit an deren Mantelfläche angeordneten Ausnehmungen aufweisenden Eindrehwerkzeugs und eine an der Peripherie der Aufnahmeöffnung angeordnete federgestützte Klinke aufweist, und wobei die Klinke bei Betätigung des Ratschenkopfs im Einschraubmodus mit einer Ausnehmung des Eindrehwerkzeugs in mitnehmenden Eingriff kommt und bei Betätigung des Ratschenkopfs im Ratschenmodus der mitnehmende Eingriff gelöst ist. Ferner wird die Aufnahmeöffnung von einer Umfassung gebildet, auf deren Innenseite die Klinke einstückig angeordnet ist. Erfindungsgemäß ist vorgesehen, dass die Umfassung in Umfangsrichtung eine Unterbrechung aufweist und somit einen biegeelastischen offenen Ring als Feder für die Klinke bildet, an dessen erstem Ende die Klinke und an dessen zweitem Ende der Betätigungsgriff angeordnet ist.

Weitere vorteilhafte Ausgestaltungen der Erfindung sind Gegenstand der Unteransprüche.

Reinigung und Sterilisation werden weiter vereinfacht, wenn Ratschenkopf und Betätigungsgriff aus oder in einem Stück gefertigt sind, und somit eine Demontage zur Reinigung und Sterilisation entfallen kann. Der Ratschenkopf kann auch separat gefertigt werden, um die Verwendung verschiedener Betätigungsgriffe zu ermöglichen.

Vorteilhafterweise umfasst der Betätigungsgriff einen Drehmomentintegrator und/oder -anzeiger und/oder -begrenzer.

Soll das Schraubwerkzeug als Ratsche üblicher Bauart zum Einsatz kommen, so ist der Betätigungsgriff vorteilhafterweise radial an einem Ende der Umfassung angeordnet. Auch hier können Ratschenkopf und Betätigungsgriff zusammen aus einem Stück gefertigt sein.

Soll das Schraubwerkzeug wie ein Schraubendreher bedient werden können, so ist der Betätigungsgriff vorteilhafterweise axial an einem Ende der Umfassung angeordnet. Auch hier ist eine einteilige Ausführung von Ratschenkopf und Betätigungsgriff denkbar.

In einer weiteren bevorzugten Ausführungsform besteht der Betätigungsgriff aus einem zumindest teilweise hohlem Rohr. So können Sensoren und Elektronik für ein Drehmomentanzeigegerät im Inneren des Betätigungsgriffs untergebracht werden, wodurch eine einfache Reinigung und Sterilisation gewährleistet ist.

Vorteilhafterweise ist das Rohr des Betätigungsgriffs unterteilt in einen Bereich, der den eigentlichen Griff bildet, und einen Schaft zwischen Ratschenkopf und diesem eigentlichem Griff. Somit kann der eigentliche Griff biegesteif und der Bereich des Schaftes als Biegebalken ausgebildet sein, wodurch sichergestellt werden kann, dass das Signal eines im Schaftbereich untergebrachten Dehnungsmessstreifens bei Betätigung des Griffs nicht verfälscht wird.

Der Schaft enthält vorteilhafterweise einen oder mehrere Dehnungsmessstreifen, die vorzugsweise im Inneren des hohlen Rohres angebracht sind.

In einer weiteren bevorzugten Ausführungsform ist der Betätigungsgriff zur Übertragung des Signals des oder der Dehnungsmessstreifen und zur Drehmomenterfassung an ein Mess-, Anzeige- und Dokumentationsmodul angeschlossen.

Der Anschluss an das Mess-, Anzeige- und Dokumentationsmodul kann dabei kabelgebunden oder drahtlos ausgeführt sein. Bei der drahtlosen Ausführung ist zur Funkübertragung des Messsignals eine entsprechende Sendeeinheit im Inneren des Rohres untergebracht. Um die Notwendigkeit einer Demontage des Betätigungsgriffs zur Reinigung und Sterilisation zu verhindern, ist das Innere des Betätigungsgriffs vorzugsweise mittels einer Verschlusseinheit vollständig verkapselt.

In einer weiteren bevorzugten Ausführungsform wird der Drehmomentverlauf während eines Eindrehvorgangs durch das Mess-, Anzeige- und Dokumentationsmodul laufend angezeigt, überwacht und dokumentiert. Dies kann auch die Überwachung und Einhaltung von Grenzwerten für das Eindrehmoment, welche vor dem Eindrehvorgang eingegeben werden, durch visuelle Darstellung sowie visuelle, akustische oder auch haptische Signale, wie beispielsweise Vibration, beinhalten. Da diese Ausführungsform grundsätzlich auch bei anders gestalteten Schraubwerkzeugen bzw. Werkzeugköpfen zum Einsatz kommen kann, wird für diese und die folgende Ausführungsform selbstständig Schutz begehrt.

Vorteilhafterweise wird der komplette Drehmomentverlauf während eines Eindrehvorgangs zu Dokumentationszwecken in einem Patientendatensatz abgespeichert. Um direkt im Anschluss an das Einsetzen eines Implantats eine qualitative Aussage treffen zu können, ob das Implantat richtig eingesetzt wurde, spricht man in der dentalen Medizin von einer sogenannten Primärstabilität. Damit ist die Torsionsbelastbarkeit gemeint, die ein eingebrachtes Implantat direkt nach dem eigentlichen Implantieren aufweist. Sie entspricht dem aufgezeichneten Drehmomentwert bei Beendigung des Eindrehvorgangs. Ein Arzt kann mit Hilfe des abgespeicherten Drehmomentverlaufs somit zu einem späteren Zeitpunkt jederzeit Rechenschaft über den sogenannten Insertionsmomentenverlauf sowie über die maximale Drehmomentsbelastung während und bei Beendigung der Insertion geben.

Im Folgenden wird ein Ausführungsbeispiel des erfindungsgemäßen Schraubwerkzeugs anhand von Zeichnungen näher erläutert.

Es zeigen:
- Fig. 1: eine Draufsicht eines erfindungsgemäßen Schraubwerkzeugs mit einem Rat- schenkopf und einem Betätigungsgriff;
- Fig. 2: eine Seitenansicht des Schraubwerkzeugs aus Fig. 1 mit einem Eindrehwerk- zeug, welches in die Aufnahmeöffnung des Ratschenkopfes eingesteckt werden kann;
- Fig. 3: eine perspektivische Ansicht des Schraubwerkzeugs aus den Fig. 1 und 2;
- Fig. 4: eine Schnittansicht der Seitenansicht des erfindungsgemäßen Schraubwerk- zeugs aus Fig. 2; und
- Fig.5: eine beispielhafte Anzeige des Mess-, Anzeige- und Dokumentationsmoduls.

Für die gesamte weitere Beschreibung gilt: Sind in einer Figur zum Zweck zeichnerischer Eindeutigkeit Bezugsziffern enthalten, aber im zugehörigen Beschreibungstext nicht erläutert, so wird auf deren Erwähnung in vorangehenden Figurenbeschreibungen Bezug genommen.

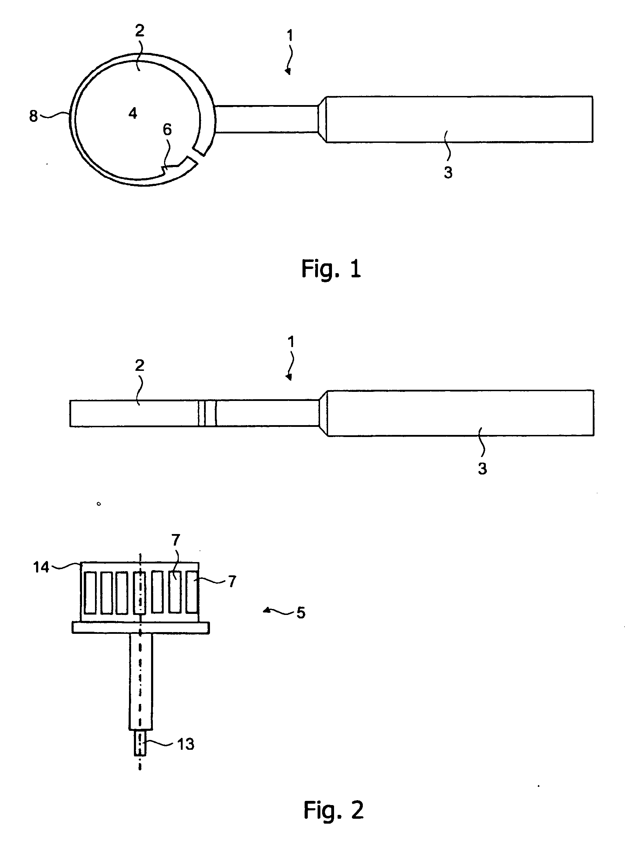

Fig. 1 zeigt eine Draufsicht eines erfindungsgemäßen Schraubwerkzeugs 1 mit einem Ratschenkopf 2 und einem Betätigungsgriff 3. Der Ratschenkopf 2 weist eine Aufnahmeöffnung 4 auf, die von einer Umfassung 8 umgeben ist. Die Umfassung 8 ist zur Aufnahme eines Eindrehwerkzeugs 5 (siehe Fig. 2) ringförmig ausgebildet und weist an einer Stelle in Umfangsrichtung eine Unterbrechung auf. Im Bereich dieser Unterbrechung ist auf einer Seite der Umfassung 8 eine Klinke 6 und auf der anderen Seite ein Betätigungsgriff 3 angeordnet. Klinke 6 und Umfassung 8 sind einstückig ausgeführt, wobei die Umfassung 8 die Federung der Klinke übernimmt. Die Federkraft wird dabei durch die Materialstärke der Umfassung 8 sowie durch die Materialwahl und somit die Werkstoffeigenschaften festgelegt. Ratschenkopf 2 und Betätigungsgriff 3 sind aus einem Stück gefertigt, um Reinigung und Sterilisation des Schraubwerkzeugs 1 zu vereinfachen. Der Ratschenkopf 2 kann jedoch auch separat gefertigt werden, um in Kombination mit verschiedenen Betätigungsgriffen 3 verwendet werden zu können.

Fig. 2 zeigt eine Seitenansicht des erfindungsgemäßen Schraubwerkzeugs 1 aus Fig. 1 mit einem ebenfalls in einer Seitenansicht gezeigten Eindrehwerkzeug 5. Das Eindrehwerkzeug 5 hat einen oberen Teil mit einer zylindrischen Handhabe, deren Mantelfläche 14 mehrere Ausnehmungen 7 aufweist. Konzentrisch zur zylindrischen Handhabe verläuft der Schaft des Eindrehwerkzeugs mit der Werkzeugklinge 13. Wird das Eindrehwerkzeug 5 in die Aufnahmeöffnung 4 des Schraubwerkzeugs 1 eingesteckt, so greift die Klinke 6 in eine der Ausnehmungen 7 ein. Wird der Betätigungsgriff 3 in Einschraubrichtung bewegt, so besteht zwischen Klinke 6 und Ausnehmung 7 ein mitnehmender Eingriff. Wird der Betätigungsgriff 3 in der Gegenrichtung bewegt, so wird die Umfassung 8 durch eine Keilwirkung der Klinke 6 aufgebogen, so dass der mitnehmende Eingriff zwischen Klinke 6 und Ausnehmung 7 gelöst wird. Die Umkehr der eingreifenden Drehrichtung erfolgt durch einfaches Umstecken des Schraubwerkzeugs 1. Das Schraubwerkzeug 1 kann für verschiedene bekannte Eindrehwerkzeuge 5 gefertigt werden, indem die Formgebung der Klinke 6 entsprechend der Ausnehmungen 7 des jeweiligen Eindrehwerkzeugs 5 angepasst wird. Die Klinke 6 kann auch derart ausgebildet sein, dass sie sowohl Links- wie auch Rechtslauf zulässt. Durch entsprechende Formgebung bzw. Werkstoffeigenschaften der Umfassung kann auch eine Drehmomentbegrenzung eingerichtet werden.

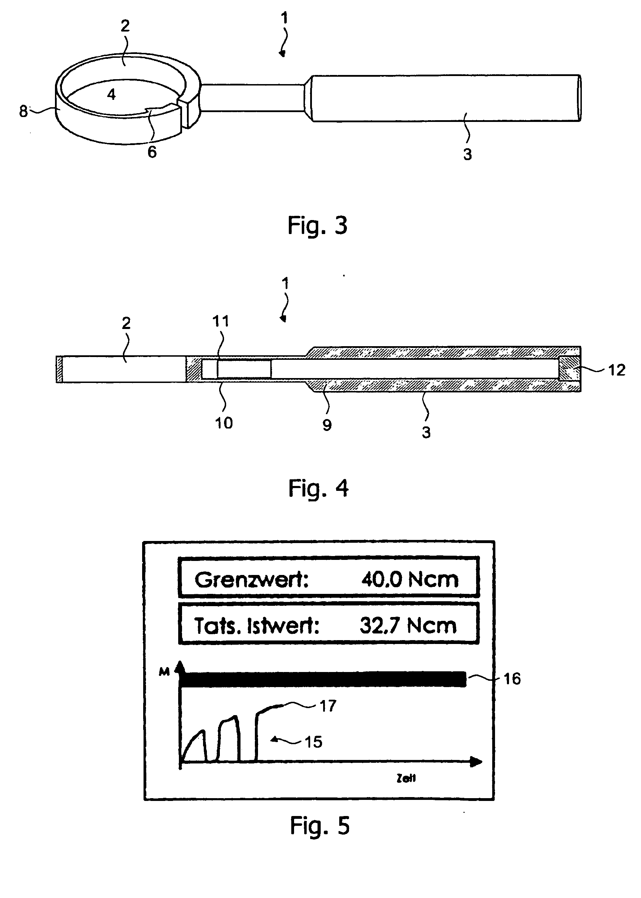

Fig. 3 zeigt eine isometrische Ansicht des bereits beschriebenen Schraubwerkzeugs aus den Fig. 1 und 2.

Fig. 4 zeigt eine Schnittansicht der Seitenansicht des Schraubwerkzeugs 1 aus der Fig. 2. Der Schnitt zeigt, dass der Betätigungsgriff 3 aus einem hohlen Rohr 9 gefertigt ist. Das Rohr 9 ist in zwei Abschnitte unterteilt. Im Bereich des Ratschenkopfes 2 befindet sich ein Schaft 10, der als dünnwandiger Biegebalken ausgebildet ist. Der darauffolgende hintere Teil des Betätigungsgriffs 3 ist dickwandiger und somit biegesteif ausgeführt. Zur Verstärkung des hinteren Teils des Betätigungsgriffs 3 können zusätzlich Versteifungsrippen oder dergleichen vorgesehen werden. Im Bereich des Schaftes 10 ist im Inneren des Rohres 9 ein Dehnungsmessstreifen 11 angebracht, um die Messung der Schaftdurchbiegung und somit des wirkenden Drehmoments während des Eindrehvorgangs zu ermöglichen. Das Signal des Dehnungsmessstreifens 11 wird über eine nicht bezeichnete ebenfalls im Inneren des Rohres 9 untergebrachte Funksendeeinheit an ein Mess-, Anzeige- und Dokumentationsmodul übertragen. Da das Schraubwerkzeug 1 zum Zwecke einer einfachen Reinigung und Sterilisation einteilig ausgeführt ist, ist das Rohr 9 des Betätigungsgriffs 3 durch eine Verschlusseinheit 12 dicht verschlossen.

Fig. 5 zeigt eine beispielhafte Anzeige des Mess-, Anzeige- und Dokumentationsmoduls. Dargestellt ist ein Beispiel eines typischen Drehmomentverlaufs 15 während des Eindrehvorgangs eines Implantats, auch Insertionsvorgang oder Insertion genannt, über der Zeit. Die Anzeige umfasst ferner die visuelle Darstellung der zuvor eingegebenen Grenzwerte 16 sowie Grenzwert und momentanen Istwert in Zahlen. Der Wechsel zwischen Einschraubmodus und Ratschenmodus ist dem Drehmomentverlauf 15 deutlich zu entnehmen. Während das Drehmoment während eines Einschraubschrittes kontinuierlich ansteigt, fällt es im Ratschenmodus auf den Wert 0 Ncm ab. Der höchste Drehmomentwert wird in der Regel am Ende der Insertion erreicht. Dieser Endwert 17 des Insertionsmomentenverlaufs gibt Aufschluss über die Torsions-Belastbarkeit direkt im Anschluss an die Insertion, der sog. Primärstabilität. Der gesamte Drehmomentverlauf wird zusammen mit den eingegebenen Grenzwerten und dem ermittelten Wert der Primärstabilität in einem Patienten-spezifischen Datensatz abgespeichert und archiviert.

## Patentansprüche

1. Schraubwerkzeug (1) mit einem Ratschenkopf (2) und einem Betätigungsgriff (3), insbesondere für den Einsatz in der Medizinaltechnik, wobei der Ratschenkopf (2) eine Aufnahmeöffnung (4) zum Einsetzen eines herkömmlichen, eine im Wesentlichen zylindrische Handhabe mit an der Mantelfläche (14) angeordneten Ausnehmungen (7) aufweisenden Eindrehwerkzeugs (5) und eine an der Peripherie der Aufnahmeöffnung (4) angeordnete federgestützte Klinke (6) aufweist, wobei die Klinke (6) bei Betätigung des Ratschenkopfs (2) im Einschraubmodus mit einer Ausnehmung (7) des Eindrehwerkzeugs (5) in mitnehmenden Eingriff kommt und bei Betätigung des Ratschenkopfs (2) im Ratschenmodus der mitnehmende Eingriff gelöst ist, und wobei die Aufnahmeöffnung (4) von einer Umfassung (8) gebildet ist, auf deren Innenseite die Klinke (6) einstückig angeordnet ist,
**dadurch gekennzeichnet, dass**
die Umfassung (8) in Umfangsrichtung eine Unterbrechung aufweist und somit einen biegeelastischen offenen Ring als Feder für die Klinke (6) bildet, an dessen erstem Ende die Klinke (6) und an dessen zweitem Ende der Betätigungsgriff (3) angeordnet ist.

2. Schraubwerkzeug (1) nach Anspruch 1,
**dadurch gekennzeichnet, dass**
Ratschenkopf (2) und Betätigungsgriff (3) in einem Stück gefertigt sind.

3. Schraubwerkzeug (1) nach einem der vorstehenden Ansprüche,
**dadurch gekennzeichnet, dass**
der Betätigungsgriff (3) einen Drehmomentintegrator und/oder -anzeiger und/oder begrenzer umfasst.

4. Schraubwerkzeug (1) nach einem der vorstehenden Ansprüche,
**dadurch gekennzeichnet, dass**
der Betätigungsgriff (3) radial am Ratschenkopf (2) als Ratschengriff üblicher Bauart angeordnet ist.

5. Schraubwerkzeug (1) nach einem der Ansprüche 1 bis 3,
**dadurch gekennzeichnet, dass**
der Betätigungsgriff (3) axial am Ratschenkopf (2) als Schraubendrehergriff angeordnet ist.

6. Schraubwerkzeug (1) nach einem der Ansprüche 3 bis 5,
**dadurch gekennzeichnet, dass**
der Betätigungsgriff (3) ein zumindest teilweise hohles Rohr (9) umfasst.

7. Schraubwerkzeug (1) nach Anspruch 6,
**dadurch gekennzeichnet, dass**
das Rohr (9) einen Schaft (10) im Bereich des Ratschenkopfs (2) aufweist, wobei der Schaft (10) als Biegebalken ausgebildet ist.

8. Schraubwerkzeug (1) nach Anspruch 7,
**dadurch gekennzeichnet, dass**
der Schaft (10) mindestens einen Dehnungsmessstreifen (11) enthält.

9. Schraubwerkzeug (1) nach Anspruch 8,
**dadurch gekennzeichnet, dass**
der Betätigungsgriff (3) zur Übertragung des Signals des/der Dehnungsmessstreifen (11) und zur Drehmomenterfassung an ein Mess-, Anzeige- und Dokumentationsmodul angeschlossen ist.

10. Schraubwerkzeug (1) nach Anspruch 9,
**dadurch gekennzeichnet, dass**
der Anschluss an das Mess-, Anzeige- und Dokumentationsmodul kabelgebunden ausgeführt ist.

11. Schraubwerkzeug (1) nach Anspruch 9,
**dadurch gekennzeichnet, dass**
der Anschluss an das Mess-, Anzeige- und Dokumentationsmodul drahtlos ausgeführt ist.

12. Schraubwerkzeug (1) nach einem der Ansprüche 8 bis 11,
**dadurch gekennzeichnet, dass**
das Innere des Betätigungsgriffs (3) mittels einer Verschlusseinheit (12) vollständig verkapselt ist.

13. Schraubwerkzeug (1) nach einem der Ansprüche 9 bis 12,
**dadurch gekennzeichnet, dass**
das Mess-, Anzeige- und Dokumentationsmodul derart ausgelegt ist, dass der Drehmomentverlauf (15) während eines Eindrehvorgangs angezeigt, überwacht und dokumentiert wird.

14. Schraubwerkzeug (1) nach einem der Ansprüche 9 bis 13,
**dadurch gekennzeichnet, dass**
das Mess-, Anzeige- und Dokumentationsmodul derart ausgelegt ist, dass der Drehmomentverlauf (15) während eines Eindrehvorgangs in einem Patientendatensatz abgespeichert wird.

15. Schraubwerkzeug (1) nach einem der vorstehenden Ansprüche,
**dadurch gekennzeichnet, dass**
der innere Durchmesser der Umfassung (8) in Achsrichtung der Aufnahmeöffnung (4) konstant ist, so dass eine Umkehr der eingreifenden Drehrichtung durch einfaches Umstecken des Schraubwerkzeugs (1) erfolgen kann.

## Claims

1. Screw tool (1) with a ratchet head (2) and an actuating handle (3), in particular for use in medical technology, wherein the ratchet head (2) has a receiving opening (4) for the insertion of a conventional screw-in tool (5) having a substantially cylindrical handle with recesses (7) arranged on the outer surface (14), and a spring-supported catch (6) arranged on the periphery of the receiving opening (4), wherein the catch (6), on actuation of the ratchet head (2) in the screw-in mode, drivingly engages in the screw-in mode with a recess (7) of the screw-in tool (5) and on actuation of the ratchet head (2) in the ratchet mode the driving engagement is released, and wherein the receiving opening (4) is formed by an enclosure (8), on the inside of which the catch (6) is integrally arranged,
**characterised in that**
the enclosure (8) has a break in circumferential direction and consequently forms a flexurally elastic open ring as spring for the catch (6), on the first end of which the catch (6) and on the second end of which the actuating handle (3) is arranged.

2. Screw tool (1) according to claim 1,
**characterised in that**
ratchet head (2) and actuating handle (3) are fabricated in one piece.

3. Screw tool (1) according to one of the preceding claims,
**characterised in that**
the actuating handle (3) comprises a torque integrator and/or indicator and/or limiter.

4. Screw tool (1) according to one of the preceding claims,
**characterised in that**
the actuating handle (3) is arranged radially on the ratchet head (2) as ratchet handle of conventional construction.

5. Screw tool (1) according to one of claims 1 to 3,
**characterised in that**
the actuating handle (3) is arranged axially on the ratchet head (2) as screw turning handle.

6. Screw tool (1) according to one of claims 3 to 5,
**characterised in that**
the actuating handle (3) comprises an at least partially hollow pipe (9).

7. Screw tool (1) according to claim 6,
**characterised in that**
the pipe (9) has a shaft (10) in the area of the ratchet head (2), wherein the shaft (10) is developed as a bending bar.

8. Screw tool (1) according to claim 7,
**characterised in that**
the shaft (10) contains at least one strain gauge (11).

9. Screw tool (1) according to claim 8,
**characterised in that**
the actuating handle (3) is connected to a measuring, display and documentation module for transferring the signal of the strain gauge(s) (11) and for recording the torque.

10. Screw tool (1) according to claim 9,
**characterised in that**
the connection to the measuring, display and documentation module is executed cable-connected.

11. Screw tool (1) according to claim 9,
**characterised in that**
the connection to the measuring, display and documentation module is executed wireless.

12. Screw tool (1) according to one of claims 8 to 11,
**characterised in that**
the inside of the actuating handle (3) is completely encapsulated by means of a sealing unit (12).

13. Screw tool (1) according to one of claims 9 to 12,
**characterised in that**
the measuring, display and documentation module is designed such that the torque history (15) during a screw-in procedure is displayed, monitored and documented.

14. Screw tool (1) according to one of claims 9 to 13,
**characterised in that**
the measuring, display and documentation module is designed such that the torque history (15) during a screw-in procedure is stored in a patient record.

15. Screw tool (1) according to one of the preceding claims,
**characterised in that**
the inner diameter of the enclosure (8) in axial direction of the receiving opening (4) is constant, so that a reversal of the engaging direction of rotation can be carried out by simply inversing the screw tool (1).

## Revendications

1. Outil de vissage (1) avec une tête à cliquet (2) et une poignée d'actionnement (3), à utiliser en particulier dans la technique médicale, étant précisé que la tête à cliquet (2) présente une ouverture de réception (4) pour l'introduction d'un outil de vissage (5) classique qui présente une manette globalement cylindrique avec des creux (7) disposés sur la surface latérale (14), et un cliquet à ressort (6) disposé sur la périphérie de l'ouverture de réception (4), que le cliquet (6), lors de l'actionnement de la tête (2) en mode de vissage, vient en prise d'entraînement avec un creux (7) de l'outil de vissage (5), tandis que lors de l'actionnement de la tête (2) en mode de cliquet, la prise d'entraînement est supprimée, et que l'ouverture de réception (4) est formée par un cerclage (8) sur le côté intérieur duquel le cliquet (6) est réalisé d'une seule pièce,
**caractérisé en ce que** le cerclage (8) présente une interruption, dans le sens circonférentiel, et forme ainsi un anneau ouvert flexible, comme ressort pour le cliquet (6), à la première extrémité duquel est disposé le cliquet (6) et à la seconde extrémité duquel est disposée la poignée d'actionnement (3).

2. Outil de vissage (1) selon la revendication 1, **caractérisé en ce que** la tête à cliquet (2) et la poignée d'actionnement (3) sont réalisées d'une seule pièce.

3. Outil de vissage (1) selon l'une des revendications précédentes, **caractérisé en ce que** la poignée d'actionnement (3) comprend un intégrateur et/ou indicateur et/ou limiteur de couple.

4. Outil de vissage (1) selon l'une des revendications précédentes, **caractérisé en ce que** la poignée d'actionnement (3) est disposée radialement sur la tête à cliquet (2), sous la forme d'une poignée à cliquet à construction courante.

5. Outil de vissage (1) selon l'une des revendications 1 à 3, **caractérisé en ce que** la poignée d'actionnement (3) est disposée axialement sur la tête à cliquet (2), sous la forme d'une poignée de tournevis.

6. Outil de vissage (1) selon l'une des revendications 3 à 5, **caractérisé en ce que** la poignée d'actionnement (3) comprend un tube (9) au moins en partie creux.

7. Outil de vissage (1) selon la revendication 6, **caractérisé en ce que** le tube (9) présente une tige (10) dans la zone de la tête à cliquet (2), la tige (10) étant conçue comme un élément flexible.

8. Outil de vissage (1) selon la revendication 7, **caractérisé en ce que** la tige (10) contient au moins une jauge extensométrique (11).

9. Outil de vissage (1) selon la revendication 8, **caractérisé en ce que** la poignée d'actionnement (3) est raccordée, pour transmettre le signal de ladite jauge extensométrique (11) et pour relever le couple, à un module de mesure, d'affichage et de documentation.

10. Outil de vissage (1) selon la revendication 9, **caractérisé en ce que** le raccordement au module de mesure, d'affichage et de documentation se fait par câble.

11. Outil de vissage (1) selon la revendication 9, **caractérisé en ce que** le raccordement au module de mesure, d'affichage et de documentation se fait sans fil.

12. Outil de vissage (1) selon l'une des revendications 8 à 11, **caractérisé en ce que** l'intérieur de la poignée d'actionnement (3) est complètement enveloppé à l'aide d'une unité de fermeture (12).

13. Outil de vissage (1) selon l'une des revendications 9 à 12, **caractérisé en ce que** le module de mesure, d'affichage et de documentation est conçu pour que la courbe de couple (15) soit affichée, surveillée et documentée pendant une opération de vissage.

14. Outil de vissage (1) selon l'une des revendications 9 à 13, **caractérisé en ce que** le module de mesure, d'affichage et de documentation est conçu pour que la courbe de couple (15) soit mise en mémoire dans un ensemble de données de patient pendant une opération de vissage.

15. Outil de vissage (1) selon l'une des revendications précédentes, **caractérisé en ce que** le diamètre intérieur du cerclage (8) est constant dans le sens axial de l'ouverture de réception (4), de sorte qu'une inversion du sens de rotation actif peut se faire grâce à un simplement retournement de l'outil de vissage (1).
